**Europäisches Patentamt**

**European Patent Office**

**Office européen des brevets**

(11) Veröffentlichungsnummer: **0 242 833 B1**

(12) **EUROPÄISCHE PATENTSCHRIFT**

(45) Veröffentlichungstag der Patentschrift: **19.02.92**

(21) Anmeldenummer: **87105790.7**

(22) Anmeldetag: **18.04.87**

(51) Int. Cl.⁵: **C07C 227/00**, C12P 13/06, C12P 13/08, C12P 13/22

(54) Verfahren zur Herstellung von L-Aminosäuren durch Transaminierung.

(30) Priorität: **24.04.86 DE 3613952**

(43) Veröffentlichungstag der Anmeldung:
**28.10.87 Patentblatt 87/44**

(45) Bekanntmachung des Hinweises auf die
Patenterteilung:
**19.02.92 Patentblatt 92/08**

(84) Benannte Vertragsstaaten:
**BE CH DE FR GB IT LI NL SE**

(56) Entgegenhaltungen:
EP-A- 0 132 999      EP-A- 0 135 846
EP-A- 0 152 275      EP-A- 0 167 058
BE-A- 902 313        DE-A- 3 427 495
FR-A- 2 557 887

CHEMIKER ZEITUNG, Band 98, Nr. 11, November 1974, Seiten 523-532; G. NESEMANN et al.: "Technische Anwendung mikrobieller Verfahren"

Handbuch der Biotechnologie, 2. Auflage, Oldenburg Verlag GmbH, 1984, S. 571

(73) Patentinhaber: **HOECHST AKTIENGESELL-SCHAFT**
**Postfach 80 03 20**
**W-6230 Frankfurt am Main 80(DE)**

(72) Erfinder: **Then, Johann, Dr.**
**Sulzbacher Weg 2**
**W-6238 Hofheim am Taunus(DE)**
Erfinder: **Deger, Hans-Matthias, Dr.**
**Am Rheingauer Weg 8**
**W-6238 Hofheim am Taunus(DE)**
Erfinder: **Wöhner, Gerhard, Dr.**
**Flörsheimer Strasse 27**
**W-6093 Flörsheim am Main(DE)**
Erfinder: **Voelskow, Hartmut, Dr.**
**Akazienstrasse 22**
**W-6234 Hattersheim am Main(DE)**

EP 0 242 833 B1

**Beschreibung**

Aminosäuren haben ein vielfältiges Anwendungsprofil. Sie finden ihren Einsatz als Supplement in der Tierernährung, als Nahrungsmitteladditiv für den Menschen oder als Bestandteil von Infusionslösungen. L-Phenylalanin findet eine weitere Anwendung als Synthesebaustein für den Süßstoff Aspartam, der aus Phenylalaninmethylester und Asparaginsäure besteht.

Die Herstellung von L-Aminosäuren durch Biotransformation mit Transaminasen ist an sich bekannt. In der Europäischen Patentanmeldung 152 275 wird insbesondere ein Verfahren zur Herstellung von Phenylalanin durch Transaminierung mit Hilfe eines genetisch modifizierten Mikroorganismus beschrieben, der sich durch Überproduktion der Aminotransferase auszeichnet. Die Transaminierungsreaktion wird bei mindestens 40 °C durchgeführt, da bei diesen höheren Temperaturen die Mikroorganismenzellen stärker permeabilisieren.

Nach der Europäischen Patentanmeldung 135 846 (US 4 518 692; US 45 25 454) erfolgt die Herstellung von L-Aminosäuren derart, daß $\alpha$-Ketosäuren mit L-Asparaginsäure in Gegenwart einer Transaminase umgesetzt werden, die aus E. coli isoliert wurde. Es entstehen die der Ketosäure entsprechende $\alpha$-Aminosäure sowie Oxalacetat aus der Asparaginsäure. Durch Entfernung von Oxalacetat aus dem Reaktionsmedium nach der Decarboxylierung, wird das Reaktionsgleichgewicht auf die Seite des Endprodukts verschoben. Oxalacetat decarboxyliert in wäßriger Lösung aufgrund seiner Instabilität. Diese Reaktion kann thermisch, chemisch oder enzymatisch beschleunigt werden.

Die Selektion und Mutation von Mikroorganismen aus der Reihe E coli., Paracoccus denitrificans, Torula, Rhodotorula und Streptomyces zur Herstellung von L-Phenylalanin aus Phenylbrenztraubensäure mit verbesserter Ausbeute wird in der Deutschen Patentanmeldung DE 3 423 936 dargestellt.

Überraschend wurde nun gefunden, daß durch einfaches Begasen der Reaktionslösung, in der die Transaminierung durchgeführt wird, nach kurzer Reaktionszeit eine Ausbeute der gewünschten Aminosäure von bis zu 100 % erzielt werden kann.

Die Erfindung betrifft somit ein Verfahren zur mikrobiellen Herstellung von L-Aminosäuren durch Transaminierung einer $\alpha$-Ketosäure mit Hilfe eines Aminogruppendonors, das dadurch gekennzeichnet ist, daß als Aminogruppendonor Asparagin, Asparaginsäure, Glutamin und Glutaminsäure einge setzt werden und die Reaktionslösung begast wird.

Im folgenden wird die Erfindung genau erläutert bzw. in den Patentansprüchen definiert.

Zahlreiche Mikroorganismen sind in der Lage, $\alpha$-Ketosäuren durch Biotransformation in L-Aminosäure umzuwandeln. Diese Mikroorganismen können erfindungsgemäß eingesetzt werden. Bevorzugt wird jedoch mit Paracoccus denitrificans DSM 65 sowie mit aus Erdbodenproben isolierten Streptomyceten gearbeitet. Beste Ergebnisse werden mit E. coli ATCC 11303 erzielt. Es ist vorteilhaft, jedoch nicht unbedingt notwendig, daß durch Selektion und Mutation, in an sich bekannter Weise, insbesondere gemäß dem Verfahren der Deutschen Patentanmeldung DE 3 423 936, gegen steigende Mengen der entsprechenden $\alpha$-Ketosäure in den Anzuchtmedien für die weiteren Arbeiten Mikroorganismen ausgewählt werden, die aufgrund ihrer Adaptation an die $\alpha$-Ketosäure die Biotransformation in besseren Ausbeuten durchführen.

Durch Selektion können bespielsweise Transaminaseaktivitäten von 100-200 $\mu$mol/min •l Kulturflüssigkeit zugrunde gelegt werden. Auf diese Weise können mit dem erfindungsgemäßen Verfahren bis zu 60 g/l $\alpha$-Ketosäure mit etwa 100 %iger Ausbeute zu der entsprechenden Aminosäure transaminiert werden.

Die Mikroorganismen werden vorteilhaft in einem für ihr Wachstum optimalen Nährmedium unter entsprechenden günstigen Temperatur- und Belüftungsbedingungen bis zu einem Feuchtgewicht von etwa 4 bis 10 g/l Nährlösung angezogen. Die für den jeweiligen Mikroorganismus günstigsten Bedingungen sind dem Fachmann entweder bekannt oder können in einfachen Vorversuchen festgestellt werden. Die Zellen werden dann in der Nährlösung oder von der Nährlösung abgetrennt zur Aminierung der Ketosäuren eingesetzt. Die Transaminierung kann mit ganzen oder auch mit aufgeschlossenen Zellen durchgeführt werden, wobei die üblichen Aufschlußmethoden angewendet werden. Aus Gründen der Arbeitserleichterung wird bevorzugt mit intakten Zellen gearbeitet. Es ist weiterhin möglich, die Mikroorganismen in fixierter Form einzusetzen. Für die Fixierung kommen die bekannten Verfahren in Betracht, vorteilhaft die Methoden nach den Deutschen Offenlegungsschriften 32 37 341 (US 4 603 111) und 32 43 591 (US 4 542 069).

Die Mikroorganismen werden in einem für die Zellen physiologischen Puffer suspendiert unter Zugabe der $\alpha$-Ketosäure und des Aminogruppendonors. Je nach Menge der Mikroorganismen kann die dem Ansatz zugegebene enzymatische Aktivität in weiten Bereichen schwanken. Zweckmäßig liegt sie zwischen 10 bis 20.000 $\mu$mol/min•l. Vorzugsweise enthält der Ansatz Zellmengen mit einer Enzymaktivität von 1500-2000 $\mu$mol/min•l.

Als Aminogruppendonor finden Aminosäuren, wie z.B. Glycin Alanin, Valin, Leucin, Anwendung, insbeondere Asparagin, Asparaginsäure, Glutamin und Glutaminsäure. Diese Aminosäuren werden in Form ihrer freien Säure oder geeigneten Salze (entsprechend dem verwendeten Medium) eingesetzt. Der Aminogruppendonator wird in äquimolaren Mengen bzw. im Überschuß zur α-Ketosäure eingesetzt. Verhältnisse von 1:1 bis 5:1, vorteilhaft 1:1 bis 2:1, haben sich bewährt.

Die Zugabe der Reaktionskomponenten zum Reaktionsansatz kann als Lösung in Wasser oder durch Zugabe der festen Substanzen gleichzeitig erfolgen. Bevorzugt ist jedoch eine gestaffelte bzw. kontinuierliche Zugabe in Mengen von 1-4,5 %, insbesondere 1,5-2 %, jeweils bezogen auf das Gewicht des Reaktionsansatzes, über einen Zeitraum von 1-90 Stunden, vorzugsweise 2-40 Stunden.

Es wird vorteilhaft bei einem pH-Wert zwischen 5 und 9, insbesondere zwischen 7 und 8,5, gearbeitet. Es ist außerdem zweckmäßig, die Transaminierung in einem Temperaturbereich von 20˚-65˚ C durchzuführen. Bei niedrigeren Temperaturen verläuft die Enzym-Reaktion zunehmend langsamer, während das Enzym bei höheren Temperaturen fortschreitend desaktiviert wird.

Die günstigste Vorgehensweise hängt von dem jeweiligen Mikroorganismus ab und kann in einfachen Vorversuchen leicht festgelegt werden.

Es hat sich besonders bewährt, die Mikroorganismen vor bzw. während der Transaminierungsreaktion zu permeabilisieren. Dies kann durch Zugabe geeigneter Agenzien, wie Toluol, Cetyltrimethylammoniumbromid, Dimethylsulfoxid etc., zum Inkubationsmedium erfolgen.

Hohe Umsatzraten in kurzer Zeit werden überraschenderweise dann erreicht, wenn die beschriebenen Reaktionsansätze, in denen die Biotransformation stattfindetm begast werden. Die Begasung erfolgt im Bereich von 0.1-15 VVm, vorteilhaft im Bereich von 0,2-3 VVm. VVm (Volume/Volume/minute) definiert den Bereich des Gasvolumendurchsatzes pro Volumen der Flüssigkeit pro Minute. Es können grundsätzlich alle Gase eingesetzt werden, die die Enzymaktivität des Mikroorganismus nicht wesentlich heruntersetzen. Geeignet sind beispielsweise Preßluft, reiner Sauerstoff, Stickstoff aber auch verschiedene Edelgase, wie Helium, Neon, Argon oder Krypton.

Aufgrund des günstigen Preises und ihrer Zugänglichkeit sind Preßluft und reiner Stickstoff bevorzugt.

Mit dem erfindungsgemäßen Verfahren können grundsätzlich alle α-Ketosäuren aminiert werden. Bevorzugt werden Aminosäuren eingesetzt, die in natürliche Proteine eingebaut werden, insbesondere solche, die in der Tabelle aufgeführt sind.

| Vorstufe, α-Ketosäure | Endprodukt, natürliche Aminosäure |
|---|---|
| Pyruvat | Alanin |
| Dimethylpyruvat | Valin |
| Isopropylpyruvat | Leucin |
| Ethylmethylpyruvat | Isoleucin |
| Hydroxypyruvat | Serin |
| Phenylpyruvat | Phenylalanin |
| 4-Hydroxyphenylpyruvat | Tyrosin |
| Indolpyruvat | Tryptophan |

Die anschließenden Beispiele dienen dazu, die vorgestellte Erfindung weiter zu illustrieren. Prozentangaben beziehen sich, wenn nicht anders angegeben, auf das Gewicht.

Beispiel 1

Escherichia coli ATTC 11303 wurde nach konventionellen Methoden kultiviert und mit N-Methyl-N-nitro-N-nitroguanidin (MNG) mutagenisiert. Die mit MNG behandelten Zellen wurden auf einen autoklavierten Agar mit folgender Zusammensetzung ausgestrichen:

| | |
|---|---|
| Fumarsäure | 5 g/l |
| Fleischextrakt | 20 g/l |
| Asparaginsäure | 20 g/l |
| $KH_2PO_4$ | 2 g/l |
| $MgSO_4{}^4 \cdot 7\,H_2O$ | 0,5 g/l |
| $CaCl_2 \cdot 2\,H_2O$ | 0,1 g/l |
| Agar | 20 g/l |

pH 7,2 wurde mit Natronlauge eingestellt.

Eine sterilfiltrierte Lösung von Phenylpyruvat wurde in den noch heißen Agar eingegossen, so daß eine Endkonzentration von 24 g/l Phenylpyruvat erreicht wurde. Die Platten wurden bei 37 °C 4 Tage inkubiert. Kolonien mit einem Durchmesser von ⟩1 mm wurden isoliert. 20 % der wachsenden Stämme hatten eine erhöhte Transaminaseaktivität im Vergleich zum Ausgangsstamm.

Die Transaminaseaktivitätsbestimmung wurde mit dem Sigma Test-kit G 0390 durchgeführt. Statt $\alpha$-Ketoglutarat wurde 12 mmol/l Phenylpyruvat-Natriumsalz eingesetzt.

Beispiel 2

Die gemäß Beispiel 1 selektierte Mutante von Escherichia coli ATCC 11303 wurde in der Nährlösung aus Beispiel 1 ohne Agar kultiviert. Sie hatte eine Transaminaseaktivität von 170 $\mu$mol/min•1 nach 20 Stunden Wachstum bei 37 °C. Die Zellen wurden abzentrifugiert, mit 50 mmol/l Phosphatpuffer pH 7,4 gewaschen und in 30 mmolarem Phosphatpuffer (pH 7,4) suspendiert, so daß die Suspension 1500 $\mu$mol/min•1 Transaminaseaktivität enthielt. 24 g/l Na-Phenylpyruvat und 20 g/l Asparaginsäure wurden in die Zellsuspension gegeben.

Nach einer Inkubationszeit von 6 Stunden bei 37 °C enthielt das Reaktionsgemisch 21 g/l L-Phenylalanin. Die Lösung wurde filtriert und durch Verdampfen des Wassers auf 1:10 konzentriert. Phenylalanin wurde bei pH 5,5 und 5 °C kristallisiert. Die qualitative und quantitative Bestimmung der Aminosäure erfolgte durch HPLC auf einer RP 18-Säule.

Beispiel 3

Gemäß Beispiel 1 erhaltenes Zellmaterial wurde in 100 ml einer Lösung aus 42 g/l Phenylpyruvat, 34 g/l Asparaginsäure und 10 mmol/l Phosphatpuffer pH 7,4 suspendiert, so daß die Enzymaktivität in der Lösung 1 500 $\mu$mol/l•min entspricht. Eine Hälfte des Reaktionsansatzes wurde bei 37 °C gerührt, während die andere Hälfte noch zusätzlich mit 1 l Preßluft pro Liter Reaktionspuffer und Minute begast wurde. Nach 4 Stunden enthielt das nicht belüftete Reaktionsgefäß 16,5 g/l Phenylalanin, während im belüfteten Gefäß 26,9 g/l gemessen wurden.

Beispiel 4

Zellen von Paracoccus denitrificans DSM 65, deren Menge einer Enzymaktivität von 100 $\mu$mol/l•min entspricht, wurde in 100 ml einer wäßrigen Lösung folgender Zusammensetzung suspendiert: 90 mmol/l Asparaginsäure, 27 $\mu$mol/l N-Cetyl-N,N,N-trimethyl-ammoniumbromid,20 mmol/l 4-Hydroxyphenylpyruvat und 30 mmol/l Phosphatpuffer (pH 7,4).

Nach 20 Stunden Inkubation bei 30 °C und einer Begasung von 0,5 VVm mit Stickstoff konnten 18 mmol/l L-Tyrosin gemessen werden.

Beispiel 5

Zellmaterial wie in Beispiel 3 wurde in 100 ml einer wäßrigen Lösung aus 90 mmol/l Asparaginsäure, 27 $\mu$mol/l N-Cetyl-N,N,N-trimethylammoniumbromid, 35 mmol/l Dimethylpyruvat und 30 mmol/l Phosphatpuffer (pH 7,4) inkubiert. Nach 20 Stunden bei 40 °C und einer Begasung von 1 VVm mit Preßluft konnten 30 mmol/l Valin gemessen werden.

Beispiel 6

Zellmaterial wie in Beispiel 3 wurde in 100 ml einer wäßrigen Lösung aus 320 mmol/l Phenylpyruvat Natriumsalz, 340 mmol/l Asparaginsäure, 10 $\mu$mol/l Toluol und 10 mmol/l Tris/HCl-Puffer (pH 7,4) inkubiert. Nach 5 Stunden bei einer Begasung von 0,5 VVm mit Sauerstoff konnten bei 37 °C ein Phenylalaningehalt von 150 mmol/l und bei 50 °C ein Phenylalaningehalt von 270 mmol/l gemessen werden.

Beispiel 7

Zellmaterial wie in Beispiel 3 wurde in 100 ml einer wäßrigen Lösung aus 30 g/l Phenylpyruvat Natriumsalz, 28 g/l Asparaginsäure und 10 mmol/l Tris/HCl-Puffer (pH 7,4) suspendiert. Die Suspension wurde bei 50 °C unter Begasung mit 0,5 VVm Preßluft inkubiert. Nach 2 Stunden konnte eine Phenylalaninkonzentration von 25 g/l gemessen werden. Nun wurden 30 g festes Phenylpyruvat Natriumsalz und 26 g

4

feste Asparaginsäure Natriumsalz pro Liter Reaktionsvolumen zudosiert. Nach weiteren 2 Stunden konnte eine Phenylalaninkonzentration von 45 g/l gemessen werden.

## Patentansprüche

1. Verfahren zur mikrobiellen Herstellung von L-Aminosäuren durch Transaminierung einer $\alpha$-Ketosäure mit Hilfe eines Aminogruppendonators in wäßriger Lösung, dadurch gekennzeichnet, daß als Aminogruppendonator Asparagin, Asparaginsäure, Glutamin und Glutaminsäure eingesetzt werden und die Reaktionslösung begast wird.

2. Verfahren nach Anspruch 1, dadurch gekennzeichnet, daß die Reaktionslösung mit 0,1-15 VVm begast wird.

3. Verfahren nach Anspruch 2, dadurch gekennzeichnet, daß die Reaktionslösung mit 0,2-3 VVm begast wird.

4. Verfahren nach einem oder mehreren der Ansprüche, 1 bis 3, dadurch gekennzeichnet, daß die Zugabe der $\alpha$-Ketosäure und des Aminogruppendonators zum Reaktiosmedium gleichzeitig gestaffelt oder kontinuierlich über einen Zeitraum von 1-90 Stunden erfolgt.

5. Verfahren nach Anspruch 4, dadurch gekennzeichnet, daß die Zugabe über 2-40 Stunden erfolgt.

6. Verfahren nach einem oder mehreren der Ansprüche 1 bis 5, dadurch gekennzeichnet, daß Aminogrup-pendonator und $\alpha$-Ketosäure im Verhältnis 1:1 bis 5:1 (mol:mol) eingesetzt werden.

7. Verfahren nach Anspruch 6 dadurch gekennzeichnet, daß Aminogruppendonator und $\alpha$-Ketosäure im Verhältnis 1:1 bis 2:1 (mol:mol) eingesetzt werden.

## Claims

1. A process for the microbial preparation of L-amino acids by transamination of an $\alpha$-keto acid by means of an amino group donor in aqueous solution, which comprises using asparagine, aspartic acid, glutamine and glutamic acid as the amino group donor and passing gas into the reaction solution.

2. The process as claimed in claim 1, wherein 0.1-15 volumes per volume per minute (VVm) of gas are passed into the reaction solution.

3. The process as claimed in claim 2, wherein 0.2-3 VVm of gas are passed into the reaction solution.

4. The process as claimed in one or more of claims 1 to 3, wherein the $\alpha$-keto acid and the amino group donor are added to the reaction medium simultaneously and staggered, or continuously over a period of 1-90 hours.

5. The process as claimed in claim 4, wherein the addition is carried out over a period of 2-40 hours.

6. The process as claimed in one or more of claim 1 to 5, wherein the amino group donor and the $\alpha$-keto acid are employed in a ratio of 1:1 to 5:1 (mol:mol).

7. The process as claimed in claim 6, wherein the amino group donor and the $\alpha$-keto acid are employed in a ratio of 1:1 to 2:1 (mol :mol).

## Revendications

1. Procédé pour préparer des L-aminoacides par voie microbienne, par transamination d'un $\alpha$-céto-acide à l'aide d'un donneur de groupe amino en solution aqueuse, caractérisé en ce qu'on utilise l'asparagine, l'acide aspartique, la glutamine et l'acide glutamique comme donneur de groupe amino et on introduit du gaz dans la solution réactionnelle.

**2.** Procédé selon la revendication 1, caractérisé en ce que l'on introduit 0,1 à 15 vvm de gaz dans la solution réactionnelle.

**3.** Procédé selon la revendication 2, caractérisé en ce que l'on introduit 0,2 à 3 vvm de gaz dans la solution réactionnelle.

**4.** Procédé selon une ou plusieurs des revendications 1 à 3, caractérisé en ce que l'on effectue l'addition de l'α-céto-acide et du donneur de groupe amino au milieu réactionnel simultanément de façon graduelle ou continue, pendant une durée de 1 à 90 heures.

**5.** Procédé selon la revendication 4, caractérisé en ce que l'on effectue l'addition en 2 à 40 heures.

**6.** Procédé selon une ou plusieurs des revendications 1 à 5, caractérisé en ce que l'on utilise le donneur de groupe amino et l'α-céto-acide en une proportion de 1:1 à 5:1 (en mole:mole).

**7.** Procédé selon la revendication 6, caractérisé en ce que l'on utilise le donneur de groupe amino et l'α-céto-acide en une proportion de 1:1 à 2:1 (en mole:mole).